# EUROPÄISCHE PATENTSCHRIFT

(11) **EP 2 756 800 B1**
(45) Veröffentlichungstag und Bekanntmachung des Hinweises auf die Patenterteilung: **29.06.2022**
(21) Anmeldenummer: 14150763.2
(22) Anmeldetag: 10.01.2014
(51) Int. Cl.: A61B 5/1455

(54) **Pulsoximetrie-Monitor**
Pulse oximetry monitor
Moniteur de pulsoximétrie

(30) Priorität: 22.01.2013 DE 202013000592 U
(43) Veröffentlichungstag der Anmeldung: 23.07.2014
(73) Patentinhaber: Envitec-Wismar GmbH, 23966 Wismar (DE)
(72) Erfinder: Schiffner, Michael, 23936 Harmshagen (DE); Scholl, Thomas, 23968 Wismar (DE); Kamps, John-Eric, 23970 Wismar (DE); Pothen, Marcus, 23564 Lübeck (DE); Sprössel, Marko, 23968 Wismar (DE)
(74) Vertreter: Haseltine Lake Kempner LLP

(56) Entgegenhaltungen:
- EP-A1- 2 564 775
- WO-A1-96/08197
- WO-A1-2010/082444
- WO-A2-2012/131546
- US-A1- 2006 030 759
- US-A1- 2006 100 530
- US-A1- 2007 068 527
- US-A1- 2011 190 611

## Beschreibung

Die vorliegende Erfindung betrifft einen Pulsoximetrie-Monitor mit einem Eingang für Signale von einem Pulsoximetrie-Sensor, einer Auswerteeinheit zur Auswertung der eingehenden Signale und einer Ausgabeeinheit.

Bei dem nicht-invasiven Messen von Blutparametern ist die Pulsoximetrie ein bekanntes und gängiges Verfahren. Mittels Pulsoximetrie-Monitoren und dazu passenden Pulsoximetrie-Sensoren wird beispielsweise mittels Infrarot- und anderen optischen Sensoren die Sauerstoffsättigung und/oder die Pulsfrequenz bei Patienten ermittelt und angezeigt. Beim Unter- oder Überschreiten von Grenzwerten kann ein Alarm ausgelöst werden. Für die Messung wird ein Pulsoximetrie-Sensor an einem Körperteil des Patienten angebracht, der Sensor strahlt Licht in das Gewebe des Patienten ein und aus dem reflektierten oder transmittierten Signal wird auf die Sauerstoffsättigung geschlossen. Typischerweise werden hierfür lichtemittierende Dioden und Photoempfänger verwendet. Körperteile, die zur Messung besonders geeignet sind, sind Finger, Zehen oder Ohren, bei denen die Photoelemente relativ eng und ohne Verkanten auf die Haut zum Anliegen gebracht werden können. Bei neugeborenen Kindern, bei denen die Sauerstoffversorgung ein kritischer Parameter ist, wird üblicherweise auch an der Hand oder am Fuß, vereinzelt auch am Daumen gemessen, da dir Finger zu klein zur Applikation eines Sensors sind.

Da die sichere Befestigung entsprechender Sensoren an dem Patienten für eine genaue Messung und Überwachung zwar notwendig aber nicht einfach ist, beschäftigen sich zahlreiche Dokumente des Standes der Technik mit Vorrichtungen, die eine sichere und insbesondere lange haltende Applikation des Sensors an dem Patienten erleichtern sollen. So ist beispielsweise aus dem US 4,685,464 ein Fingersensor bekannt, bei dem die Sensorelemente an einem Träger befestigt sind, der ähnlich einer Wäscheklammer aus zwei Schenkeln besteht, die über eine Schenkelfeder miteinander verbunden sind, um die Sensorelemente in ihrer Position am Finger zu halten. In der EP-A-0 481 612 ist ein Fingersensor beschrieben, bei dem der zu messende Finger auf eine Aufnahme gelegt wird, die das erste Sensorelement aufweist, und anschließend der zweite Sensor mittels eines Haftstreifens an dem Finger und der Aufnahme befestigt wird. Weitere Sensoraufbauten, die ein sicheres Befestigen eines Pulsoximetrie-Sensors am Patienten sicherstellen sollen, sind in der DE 44 29 845 C1 beschrieben.

Trotz aller Bemühungen, Pulsoximetrie-Sensoren sicher und lange haltend an einem Patienten zu befestigen, kommt es in der Praxis immer wieder zu Problemen durch Bewegungen des Patienten. Hierdurch können die Sensoren in ihrer Lage entweder soweit verändert werden, dass kein zuverlässiges Messsignal mehr erhalten werden kann oder die Sensoren können sogar vollständig von dem Körperteil, an dem sich appliziert waren, abfallen oder abgestreift werden. Dementsprechend schlägt die US 2013/0006076 A1 die Bestimmung eines Sensor-Aus-Signals vor, um automatisch ein Verschieben oder Abfallen des Sensors vom Applikationsort zu erfassen und gegebenenfalls anzuzeigen. Die DE 199 55 082 A1 schlägt vor, einen Pulsoximetrie-Sensor zusätzlich mit einem Bewegungssensor auszustatten, so dass bei detektierter Bewegung eine Alarmunterdrückung wegen gestörter Sauerstoffsättigungsmessung erfolgen kann.

Der beschriebene Stand der Technik bemühte sich bislang nur darum, Pulsoximetrie-Sensoren möglichst sicher am Patienten zu befestigen und eine Störung dieser Befestigung zu erfassen und anzuzeigen. Die Bemühung um eine möglichst sichere Befestigung, beispielsweise durch ein Anklammern mittels einer Feder wie in US 4,685,464 vorgeschlagen, führt jedoch zu dem Problem, dass bei längeren Messzeiten beispielsweise Quetschungen und Durchblutungsstörungen am Applikationsort auftreten können. Außerdem können beispielsweise bei Verwendung von Haftstreifen sowie durch das permanente Durchstrahlen des Gewebes Irritationen am Applikationsort bis hin zu punktuellen Hautverbrennungen auftreten. Es ist daher wichtig, dass der Applikationsort eines Pulsoximetrie-Sensors in regelmäßigen Zeitabständen gewechselt wird.

Im klinischen Betrieb ist es für die Pflegekräfte schwierig, zusätzlich zur Überwachung des Patienten auch noch in regelmäßigen Zeitabständen den Applikationsort eines Pulsoximetrie-Sensors zu ändern, so dass das Problem besteht, dass empfohlene Zeiten bis zum Wechsel des Applikationsorts überschritten oder ein Wechsel ganz vergessen wird. Zur Lösung dieses Problems schlägt die vorliegende Erfindung vor, bekannte Pulsoximetrie-Monitore mit einer Zeitmesseinheit zu versehen, die die Zeit von der korrekten Applikation eines Sensors bis um Ende der Applikation automatisch misst. Nach Verstreichen einer voreingestellten Zeit kann dann ein Signal die Notwendigkeit des Wechsels des Applikationsorts anzeigen. Hierdurch wird das Risiko, dass ein notwendiger Wechsel des Applikationsorts nach zu langer Zeit oder sogar gar nicht erfolgt, vermindert.

Die US 2007/068527 A1 offenbart einen Pulsoximetrie-Monitor mit den Merkmalen des Oberbegriffs des Anspruchs 1.

Die US 2011/190611 A1 offenbart Verfahren und Systeme zur Wiederbelebung eines Säuglings, die durch Titration von zusätzlichen Sauerstoffkonzentrationen gesunde Blutsauerstoffsättigungswerte im Säugling aufrechterhalten.

Die EP2564775 A1, welche zum Stand der Technik gemäß Artikel 54(3) EPÜ zählt, offenbart ein Verfahren zum Steuern der Sensorplatzierungszeit, eine physiologische Messvorrichtung und ein Sensor- und Computerprogrammprodukt für eine physiologische Messvorrichtung.

Die vorliegende Erfindung ist in den Ansprüchen definiert.

Geeignete Pulsoximetrie-Monitore und zugehörige Pulsoximetrie-Sensoren sind dem Fachmann bekannt und kommerziell erhältlich.

Die Erfindung sieht vor, dass der Zeitraum, in dem der Pulsoximetrie-Sensor am Patienten appliziert ist, vom Pulsoximetrie-Monitor automatisch erfasst wird. Die Erfassung erfolgt durch den Erhalt eines "Ein"-Signals. Ab Erhalt eines ersten "Ein"-Signals beginnt die Zeitmessung und läuft solange weiter, bis ein "Aus"-Signal erhalten wird. Sofern in diesem Zeitraum weitere "Ein"-Signale oder ein kontinuierliches "Ein"-Signal erhalten wird, beeinflusst dies die Zeitmessung nicht. Die Messung des Zeitraums erfolgt also immer vom Erhalt eines ersten "Ein"-Signals bis zum Erhalt des darauffolgenden ersten "Aus"-Signals.

Unter "Ein"-Signal bzw. "Aus"-Signal wird vorliegend ein Signal verstanden, das der Zeitmesseinheit signalisiert, ob der Pulsoximetrie-Sensor am Patienten appliziert ist. Bei Applikation erfolgt das "Ein"-Signal, das Ende der Applikation, also das Entfernen des Pulsoximetrie-Sensors vom Patienten, löst das "Aus"-Signal aus. Im Bereich der SpO₂-Messung hat sich hierfür auch der Begriff "Probe on" bzw. "Probe off" etabliert.

Die notwendigen "Ein"-Signale und "Aus"-Signale können in beliebiger Weise erzeugt werden. Eine Möglichkeit besteht darin, dass vom Bediener eine entsprechende Taste oder ein anderer Signalgeber betätigt wird. Vorteilhaft ist es jedoch, wenn die Signale automatisch erzeugt werden. Hierzu kann beispielsweise ein entsprechender zusätzlicher Sensor an dem Pulsoximetrie-Sensor vorgesehen sein. Um hierfür notwendige weiter konstruktive Ausgestaltungen bekannter Pulsoximetrie-Sensoren zu vermeiden, ist es jedoch am vorteilhaftesten, wenn der Pulsoximetrie-Monitor zusätzlich eine Erkennungseinheit (8) umfasst, die anhand des am Eingang (2) anliegenden Signals (3) entweder ein "Ein"-Signal oder ein "Aus"-Signal für die Zeitmesseinheit (7) generiert. Dies kann beispielsweise dadurch geschehen, dass kontinuierlich das vom Detektor des Pulsoximetrie-Sensors eingehende Signal überwacht wird, und die Erkennungseinheit anhand dieses Signals automatisch einen solchen Wert erkennt, aus dem auf die Applikation des Pulsoximetrie-Sensors an einem Patienten geschlossen werden kann. Der Erhalt eines solchen Messwerts löst dann das "Ein"-Signal aus. Der Abbruch des Messsignals führt dann dementsprechend zum Generieren des "Aus"-Signals.

Der Zeitraum, der seit Erhalt des ersten "Ein"-Signals von der Zeitmesseinheit gemessen wird, wird vorzugsweise mittels einer Ausgabeeinheit (9) dem Bedienpersonal angezeigt, wobei die Ausgabe beispielsweise optisch und/oder akustisch erfolgen kann. In einer weiter bevorzugten Ausführungsform handelt es sich bei der Ausgabeeinheit um eine optische Anzeige. Diese Anzeige kann beispielsweise in das bereits vorhandene Display eines Pulsoximetrie-Monitors integriert werden.

Da eine exakte Anzeige des Zeitraums beispielsweise in Form von Stunden, Minuten und gegebenenfalls sogar Sekunden bei der Vielzahl von Daten, die bei der Überwachung eines Patienten anfallen können, zusätzlich verwirrend sein kann, hat es sich als vorteilhaft herausgestellt, wenn die Ausgabeeinheit nur nach jeder vollen abgelaufenen Stunde ab Erhalt des "Ein"-Signals eine Ausgabe erzeugt. So kann beispielsweise innerhalb der ersten Stunde die Anzeige "0 h" sein. Innerhalb der zweiten Stunde ist die Anzeige "1 h" innerhalb der dritten Stunde "2 h", etc.

Die maximale Applikationsdauer des Pulsoximetrie-Sensors kann variieren und hängt beispielsweise vom Sensortyp, Patient und Applikationsort ab. Bei Neonaten sollte die Applikationsdauer beispielsweise 4 Stunden nicht überschreiten, wohingegen bei Erwachsenen Applikationsdauern von bis zu 8 Stunden möglich sind. Auch die Art der Befestigung des Sensors am Patienten (Klammer, Heftstreifen, etc.) kann einen Einfluss auf die mögliche Applikationsdauer haben. In einer Ausführungsform sieht die vorliegende Erfindung daher vor, dass die Ausgabeeinheit nach Ablauf einer vorgewählten Zeit ab Erhalt des "Ein"-Signals zusätzlich ein Alarmsignal ausgibt. Hierzu verfügt der erfindungsgemäße Pulsoximetrie-Monitor zusätzlich vorzugsweise über eine Zeitvorwahleingabeeinheit, beispielsweise in Form eines entsprechenden Schalters. Wird keine Zeit vorgewählt, wird das Alarmsignal vorzugsweise nach einer fest voreingestellten Zeit von beispielsweise 4 Stunden ausgegeben.

Wenn beispielsweise nach Ablauf von 4 Stunden (z.B. bei der Messung an Neonaten) der Applikationsort eines Pulsoximetrie-Sensors geändert werden soll, gibt die Ausgabeeinheit nach Ablauf von vier Stunden ab Erhalt des "Ein"-Signals zusätzlich ein Alarmsignal aus. Ein solches Alarmsignal kann akustisch erfolgen oder beispielsweise dadurch, dass ab Beginn der fünften Stunde die Anzeige "4 h" sowie die Anzeigen in den nachfolgenden Stunden, also "5 h", "6 h" etc. in der optischen Anzeige blinkt. Auch ein Wechsel der Anzeigefarbe ist möglich.

Um möglichst wenig verschiedene Informationen an das Bedienpersonal zu geben und die Bedienung des Pulsoximetrie-Monitors damit insgesamt zu vereinfachen, hat es sich als vorteilhaft erwiesen, wenn die Ausgabeeinheit über nur 9 verschiedene Zustände verfügt, die beispielsweise über die optische Anzeige für das Bedienpersonal visualisiert werden. Dementsprechend kann beispielsweise wie vorstehend beschrieben innerhalb der ersten Stunde nach Erhalt des "Ein"-Signals in der optischen Anzeige "0 h" angezeigt werden. Nach Ablauf der ersten Stunde und innerhalb der zweiten Stunde wird "1 h" angezeigt, etc. Nach Ablauf der vierten Stunde und innerhalb der fünften Stunde wird "4 h" beispielsweise blinkend angezeigt. Die Anzeige bleibt dann innerhalb der nächsten Stunden jeweils mit "5 h", "6 h" und "7 h" blinkend. Nach Ablauf der achten Stunde zeigt die optische Anzeige dann ab Beginn der neunten Stunde ">8 h" weiterhin blinkend bis zum Erhalt des "Aus"-Signals oder bis zum Abschalten des Pulsoximetrie-Monitors. Die Anzeige verändert sich in den darauffolgenden Stunden also nicht mehr, sondern zeigt ab Beginn der neunten Stunde konstant ">8 h" an, vorzugsweise blinkend.

Nach Wechsel des Applikationsorts des Pulsoximetrie-Sensors beginnt die Zeitmessung vorzugsweise neu bei 0. Dementsprechend wird die Zeitmesseinheit (7) vorzugsweise bei Erhalt eines "Aus"-Signals auf 0 zurückgesetzt. Ebenfalls beim Einschalten des Pulsoximetrie-Monitors kann die Zeitmesseinheit (7) auf 0 zurückgesetzt werden, damit anschließend bei der Applikation des Pulsoximetrie-Sensors an einen Patienten das erfolgende "Ein"-Signal den Beginn einer neuen Zeitmessung auslösen kann.

In einer weiteren Ausführungsform der vorliegenden Erfindung ist es vorgesehen, dass die Ausgabeeinheit (9) des Pulsoximetrie-Monitors abschaltbar ist. Dies kann beispielsweise dann von Vorteil sein, wenn die Messung des Zeitraums der Applikation des Pulsoximetrie-Sensors auf andere Weise erfolgt und damit die zusätzliche Anzeige des Applikationszeitraums am Pulsoximetrie-Monitor selbst nicht benötigt wird. In diesem Fall sollte trotzdem die Zeitmessung in der Zeitmesseinheit (7) unabhängig von dem ein- oder ausgeschalteten Zustand der Ausgabeeinheit (9) laufen. Dies ist insbesondere in dem Fall vorteilhaft, wenn der Pulsoximetrie-Monitor zusätzlich eine Speichereinheit (1) zur Speicherung der Zeit zwischen Erhalt eines "Ein"-Signals und des darauffolgenden "Aus"-Signals umfasst. Dies ermöglicht eine anzeigeunabhängige spätere Auswertung des Applikationszeitraums. Diese Ausführungsform ist insbesondere dann vorteilhaft, wenn nicht nur ein Applikationszeitraum in der Speichereinheit gespeichert wird, sondern über einen vorgegebenen größeren Zeitraum von beispielsweise einem Tag, einer Woche, einem Monat oder ohne Zeitbegrenzung alle Applikationszeiträume.

Für eine zusätzliche Optimierung der Auswertung kann es zudem vorteilhaft sein, wenn die Speichereinheit zusätzlich speichert, wann innerhalb eines Zeitraums zwischen Erhalt eines "Ein"-Signals und Erhalt des darauffolgenden "Aus"-Signals die Ausgabeeinheit an-oder abgeschaltet ist. Hierdurch lässt sich die Effektivität der Ausgabe de Applikationszeitraums überwachen und gegebenenfalls verbessern, beispielsweise durch Zuschaltung einer zusätzlichen akustischen Ausgabe.

## Patentansprüche

1. Pulsoximetrie-Monitor (1) mit einem Eingang (2) für Signale (3) von einem Pulsoximetrie-Sensor (4), einer Auswerteeinheit (5) zur Auswertung der eingehenden Signale (3), und einer Ausgabeeinheit (9),
wobei die Signale (3) ein "Ein"-Signal und ein "Aus"-Signal umfassen, wobei das "Ein"-Signal signalisiert, dass der Pulsoximetrie-Sensor (4) an einem Patienten appliziert ist, und das "Aus"-Signal signalisiert, dass der Pulsoximetrie-Sensor (4) von dem Patienten entfernt ist,
wobei die Auswerteeinheit (5) eine Zeitmesseinheit (7) umfasst, die die Zeit ab Erhalt eines "Ein"-Signals bis zum Erhalt eines "Aus"-Signals misst, und
wobei es sich bei der Ausgabeeinheit (9) um eine optische Anzeige handelt,
**dadurch gekennzeichnet dass** die optische Anzeige innerhalb jeder abgelaufenen Stunde nach Erhalt des "Ein"-Signals einen numerischen Wert anzeigt, wie viele Stunden abgelaufen sind, und
dass die optische Anzeige nach Ablauf einer vorbestimmten Anzahl von Stunden nach Erhalt des "Ein"-Signals, bis zum Erhalt des "Aus"-Signals oder bis zum Abschalten des Pulsoximetrie-Monitors, den angezeigten numerischen Wert nicht ändert.

2. Pulsoximetrie-Monitor gemäß Anspruch 1, wobei die Auswerteeinheit (5) zusätzlich eine Erkennungseinheit (8) umfasst, die konfiguriert ist, in Abhängigkeit von dem am Eingang (2) anliegenden Signal (3), "Ein"-Signale und "Aus"-Signale zu erkennen, und die weiterhin konfiguriert ist, "Ein"-Signale und "Aus"-Signale an die Zeitmesseinheit (7) zu kommunizieren.

3. Pulsoximetrie-Monitor gemäß Anspruch 1 oder 2, wobei die Ausgabeeinheit (9) zusätzlich ein Alarmsignal ausgibt, wenn die Auswerteeinheit (5) feststellt, dass der Zeitraum ab Erhalt des "Ein"-Signals bis zum Erhalt des darauffolgenden "Aus"-Signals eine vorgewählte Zeit überschreitet.

4. Pulsoximetrie-Monitor gemäß einem der vorhergehenden Ansprüche, wobei die Zeitmesseinheit (7) auf 0 zurückgesetzt wird nach Erhalt eines "Aus"-Signals.

5. Pulsoximetrie-Monitor gemäß einem der vorhergehenden Ansprüche, wobei die Zeitmesseinheit (7) auf 0 zurückgesetzt wird wenn der Pulsoximetrie-Monitor eingeschaltet wird.

6. Pulsoximetrie-Monitor gemäß einem der Ansprüche 1 bis 5, wobei die Ausgabeeinheit (9) abschaltbar ist.

7. Pulsoximetrie-Monitor gemäß Anspruch 6 wobei die Zeitmessung in der Zeitmesseinheit (7) unabhängig von dem ein- oder ausgeschalteten Zustand der Ausgabeeinheit (9) läuft.

8. Pulsoximetrie-Monitor gemäß einem der vorhergehenden Ansprüche, zusätzlich umfassend eine Speichereinheit (10) zur Speicherung der Zeitdauer zwischen Erhalt eines "Ein"-Signals und Erhalt des darauffolgenden "Aus"-Signals.

9. Pulsoximetrie-Monitor gemäß Anspruch 8, wobei die Speichereinheit (10) zusätzlich speichert, wann innerhalb eines Zeitraums zwischen Erhalt eines "Ein"-Signals und Erhalt des darauffolgenden "Aus"-Signals die Ausgabeeinheit (9) an- oder abgeschaltet ist.

## Claims

1. A pulse oximetry monitor (1) having an input (2) for signals (3) from a pulse oximetry sensor (4), an evaluation unit (5) for evaluating the incoming signals (3), and an output unit (9),
wherein the signals (3) comprise an "on" signal and an "off" signal, wherein the "on" signal signals that the pulse oximetry sensor (4) is applied to a patient, and the "off" signal signals that the pulse oximetry sensor (4) has been removed from the patient,
wherein the evaluation unit (5) comprises a time measurement unit (7) that measures the time from receiving an "on" signal to receiving an "off" signal, and
wherein the output unit (9) is a visual display,
**characterised in that** the visual display displays a numerical value of how many hours have elapsed within each hour elapsed after receiving the "on" signal, and
**in that** the visual display does not change the displayed numerical value after a predetermined number of hours have elapsed after receiving the "on" signal, until receiving the "off" signal or until the pulse oximetry monitor is switched off.

2. The pulse oximetry monitor according to claim 1, wherein the evaluation unit (5) also comprises a detection unit (8) that is configured to detect "on" signals and "off" signals depending on the signal (3) applied to the input (2), and is further configured to communicate "on" signals and "off" signals to the time measurement unit (7).

3. The pulse oximetry monitor according to claim 1 or 2, wherein the output unit (9) also outputs an alarm signal when the evaluation unit (5) determines that the time period from receiving the "on" signal until receiving the subsequent "off" signal exceeds a preselected time.

4. The pulse oximetry monitor according to one of the preceding claims, wherein the time measurement unit (7) is reset to 0 after receiving an "off" signal.

5. The pulse oximetry monitor according to one of the preceding claims, wherein the time measurement unit (7) is reset to 0 when the pulse oximetry monitor is switched on.

6. The pulse oximetry monitor according to one of claims 1 to 5, wherein the output unit (9) can be switched off.

7. The pulse oximetry monitor according to claim 6, wherein the time measurement in the time measurement unit (7) runs independently of the switched-on or switched-off state of the output unit (9).

8. The pulse oximetry monitor according to one of the preceding claims, also comprising a memory unit (10) for storing the length of time between receiving an "on" signal and receiving the subsequent "off" signal.

9. The pulse oximetry monitor according to claim 8, wherein the memory unit (10) also stores when the output unit (9) is switched on or off within a time period between receiving an "on" signal and receiving the subsequent "off" signal.

## Revendications

1. Moniteur de pulsoxymétrie (1) comprenant une entrée (2) pour des signaux (3) provenant d'un capteur de pulsoxymétrie (4), une unité d'évaluation (5) pour évaluer les signaux entrant (3) et une unité de sortie (9),
dans lequel les signaux (3) comprennent un signal « marche » et un signal « arrêt », dans lequel le signal « marche » signale que le capteur de pulsoxymétrie (4) est appliqué sur un patient et le signal « arrêt » signale que le capteur de pulsoxymétrie (4) est retiré du patient,
dans lequel l'unité d'évaluation (5) comprend une unité de mesure du temps (7) laquelle mesure le temps à partir de la réception d'un signal « marche » jusqu'à la réception d'un signal « arrêt », et
dans lequel l'unité de sortie (9) est un affichage optique,
**caractérisé en ce que** l'affichage optique affiche, dans chaque heure écoulée après la réception du signal « marche », une valeur numérique indiquant le nombre d'heures écoulées, et
que l'affichage optique ne modifie pas la valeur numérique affichée une fois qu'un nombre prédéfini d'heures sont écoulées après la réception du signal « marche », jusqu'à la réception du signal « arrêt » ou jusqu'à l'arrêt du moniteur de pulsoxymétrie.

2. Moniteur de pulsoxymétrie selon la revendication 1, dans lequel l'unité d'évaluation (5) comprend en outre une unité de détection (8), laquelle est conçue pour détecter des signaux « marche » et des signaux « arrêt » en fonction du signal (3) se trouvant au niveau de l'entrée (2) et laquelle est en outre conçue pour communiquer les signaux « marche » et les signaux « arrêt » à l'unité de mesure de temps (7).

3. Moniteur de pulsoxymétrie selon la revendication 1 ou 2, dans lequel l'unité de sortie (9) émet en outre un signal d'alarme lorsque l'unité d'évaluation (5) détermine que l'intervalle à partir de la réception du signal « marche » jusqu'à la réception du signal « arrêt » suivant est supérieur à un temps présélectionné.

4. Moniteur de pulsoxymétrie selon l'une quelconque des revendications précédentes, dans lequel l'unité de mesure de temps (7) est réinitialisée sur 0 après la réception d'un signal « arrêt ».

5. Moniteur de pulsoxymétrie selon l'une quelconque des revendications précédentes, dans lequel l'unité de mesure de temps (7) est réinitialisée sur 0 lorsque le moniteur de pulsoxymétrie est allumé.

6. Moniteur de pulsoxymétrie selon l'une quelconque des revendications 1 à 5, dans lequel l'unité de sortie (9) peut être éteinte.

7. Moniteur de pulsoxymétrie selon la revendication 6, dans lequel la mesure du temps dans l'unité de mesure de temps (7) s'effectue indépendamment du fait que l'unité de sortie (9) soit allumée ou éteinte.

8. Moniteur de pulsoxymétrie selon l'une quelconque des revendications précédentes, comprenant en outre une unité de mémoire (10) pour mémoriser la durée entre la réception d'un signal « marche » et la réception du signal « arrêt » suivant.

9. Moniteur de pulsoxymétrie selon la revendication 8, dans lequel l'unité de mémoire (10) mémorise en outre le moment où dans l'intervalle entre la réception d'un signal « marche » et la réception du signal « arrêt » suivant, l'unité de sortie (9) est allumée ou éteinte.
